Europäisches Patentamt

European Patent Office

Office européen des brevets

(19)

(11) Publication number: **0 089 605**
**B1**

(12) # EUROPEAN PATENT SPECIFICATION

(45) Date of publication of patent specification: **06.08.86**

(51) Int. Cl.⁴: **A 61 B 19/00, B 08 B 9/00**

(21) Application number: **83102555.6**

(22) Date of filing: **15.03.83**

(54) Washing apparatus for an endoscope.

(30) Priority: **16.03.82 JP 41311/82**
**16.03.82 JP 41312/82**

(43) Date of publication of application:
**28.09.83 Bulletin 83/39**

(45) Publication of the grant of the patent:
**06.08.86 Bulletin 86/32**

(84) Designated Contracting States:
**AT BE CH DE FR GB IT LI NL SE**

(56) References cited:
**DE-A-2 947 576**
**FR-A-2 462 897**

(73) Proprietor: **OLYMPUS OPTICAL CO., LTD.**
**43-2, 2-chome, Hatagaya Shibuya-ku**
**Tokyo 151 (JP)**

(72) Inventor: **Ogasawara, Tadahiko**
**Ishikawa-ryo 2544 Ishikawa-machi**
**Hachioji-shi Tokyo (JP)**

(74) Representative: **Kuhnen, Wacker & Partner**
**Schneggstrasse 3-5 Postfach 1729**
**D-8050 Freising (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a washing apparatus for an endoscope, which detects a leakage portion of the endoscope before the endoscope is washed and disinfected and which prevents entrance of a washing liquid and a disinfectant inside the endoscope while the endoscope is being washed and disinfected.

From DE—A—2 947 576 a washing apparatus for an endoscope is known, wherein an elongated insertion section of the endoscope is coiled into the washing tank in a spiral shape, and a washing liquid is sprayed from a rotary nozzle onto the endoscope, thereby washing the endoscope. After washing, a disinfectant is supplied to the washing tank to disinfect the endoscope.

Generally, when washing and disinfecting the endoscope, if either the washing liquid or the disinfectant enters inside the endoscope, the endoscope is damaged. Since the endoscope as a whole is required to be washed, the possibility exists that liquid may enter and damage the endoscope.

It is therefore an object of the present invention to provide a washing apparatus for an endoscope, which detects a leakage portion of the endoscope before it is washed and disinfected, thereby preventing damage to the endoscope.

Solution of this object is achieved by the characterizing features of claim 1.

According to the present invention a washing apparatus for an endoscope is provided, which supplies a gas into the endoscope while it is being washed and disinfected, thereby preventing ingress of a washing liquid or a disinfectant and hence preventing damage to the endoscope.

This invention can be more fully understood from the following detailed description when taken in conjunction with the accompanying drawings, in which:

Fig. 1 is a perspective view showing the overall outer appearance of a washing apparatus according to an embodiment of the present invention;

Fig. 2 is a plan view showing the internal structure of a washing tank of the apparatus shown in Fig. 1;

Fig. 3 is a schematic view showing a piping system including tanks, valves and pumps;

Fig. 4 is a schematic view showing a main control panel;

Fig. 5 is a schematic view showing a sub-control panel;

Fig. 6 and 7 are timing charts for explaining the mode of operation of the washing apparatus shown in Fig. 1; and

Fig. 8 is a plan view showing a modification wherein an endoscope is placed in a washing apparatus.

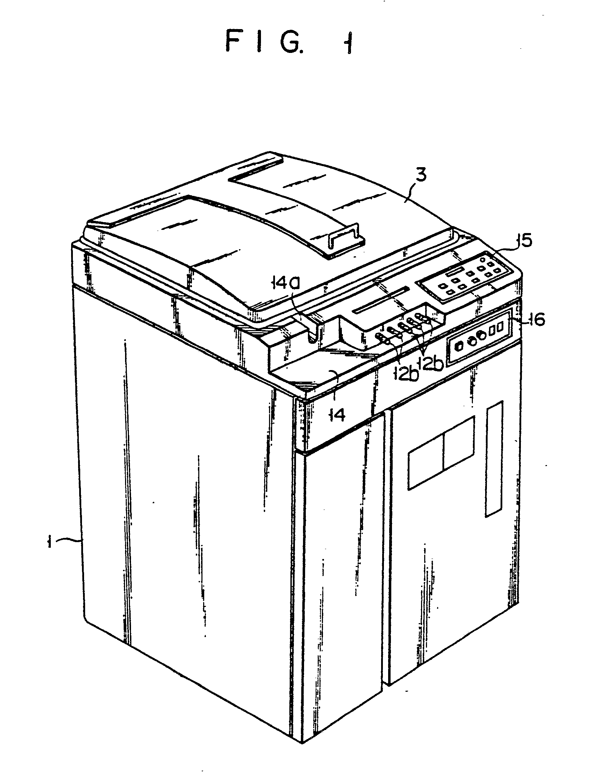

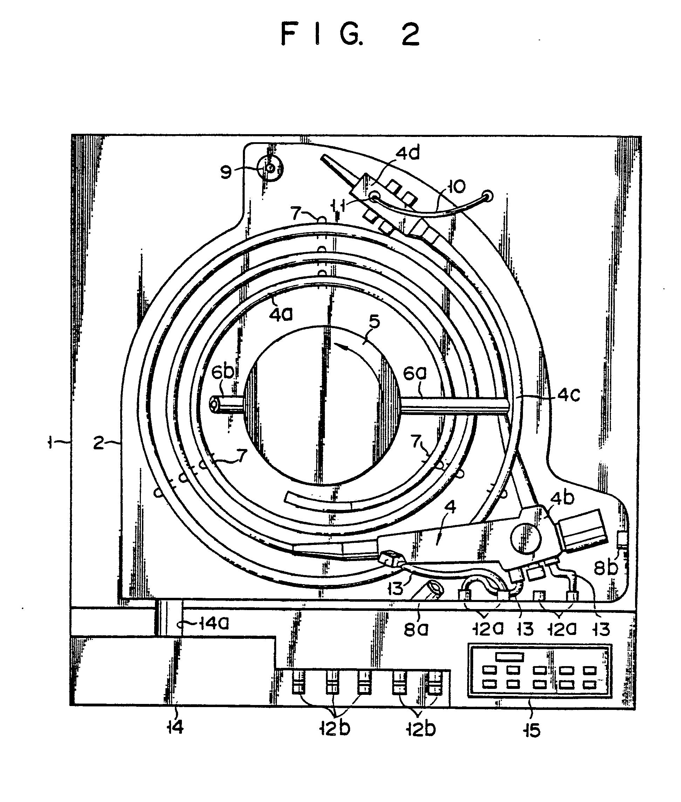

Fig. 1 shows the outer appearance of a washing apparatus for an endoscope according to an embodiment of the present invention. The washing apparatus has a main body 1, a washing tank 2 disposed inside the body 1 (as shown in Fig. 2), and a cover 3 which covers the upper opening of the washing tank 2 so as to be free to open.

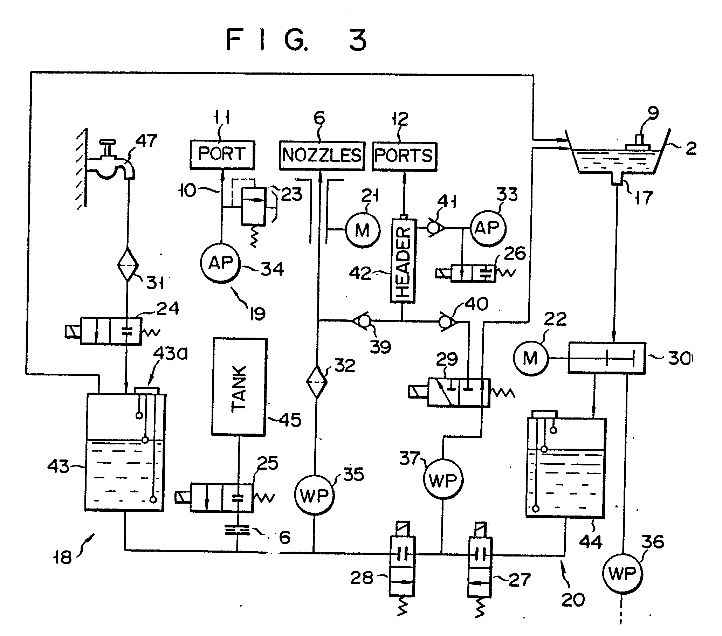

Fig. 2 shows the inside of the washing tank 2, and Fig. 3 schematically shows the pipe system for connecting the tanks, pumps, motors and valves. A rotary body 5 is rotatably mounted substantially at the center of the washing tank 2 so as to have a rotating shaft thereof disposed perpendicularly. The rotary body 5 is rotated by a motor 21. A nozzle 6a is mounted on the upper portion of the rotary body 5 so as to be able to extend and retract horizontally. The upper nozzle 6a extends near the outer peripheral portion of the washing tank 2 by the pressure of the liquid supplied to the rotary body 5 and sprays a liquid downward. A lower nozzle 6b is mounted on the lower portions of the rotary body 5 so as to spray the liquid upward at the inner portion of the washing tank 2.

A shelf 7 is fixed in the washing tank 2 so as to be disposed above the lower nozzle 6b and below the upper nozzle 6a, and an endoscope 4 is placed thereon. The endoscope 4 has an elongated insertion section 4a, a control section 4b and a light guide cable 4c, and is constructed in a liquid-tight manner as a whole. The endoscope 4 as a whole can be placed in the washing tank 2 and can be washed and disinfected. The elongated insertion section 4a and the light guide cable 4c are coiled around the rotary body 5 in opposite directions. The control section 4b is placed on a shelf (not shown) for the control section.

Stationary nozzles 8a and 8b are disposed in the vicinity of the control section 4b placed in the washing tank 2. The stationary nozzles 8a and 8b spray the liquid to the control section 4b of the endoscope 4. A float switch 9 is arranged in the washing tank 2 and is operated when the level of a liquid such as a disinfectant reaches a predetermined level. A connector 4d is mounted at the distal end of the light guide cable 4c of the endoscope 4 so as to connect a light source (not shown) and the light guide cable 4c. A pipe 10 connects an inlet port 11 formed in the connector 4d and an air pump 34 disposed under the washing tank 2 so as to supply air from the air pump 34 to the inside of the endoscope 4. A part of the pipe 10 is disposed in the vicinity of the connector 4d in the washing tank 2. This port 11 supplies compressed air from the air pump 34 to the inside of the endoscope 4 through the pipe 10. A plurality of supply ports 12a (four in this embodiment) are formed extending from the side surface of the washing tank 2 in the vicinity of the control section 4b. A washing liquid from a washing liquid source or a disinfectant from a disinfectant source is supplied to the supply ports 12a. The supply ports 12a are connected to connecting ports for channels such as a forceps channel, an air supply channel, a water supply channel and a $CO_2$ gas channel through pipes 13, respectively. The washing liquid or the disinfectant is supplied through these connecting ports to the channels of the endoscope 4.

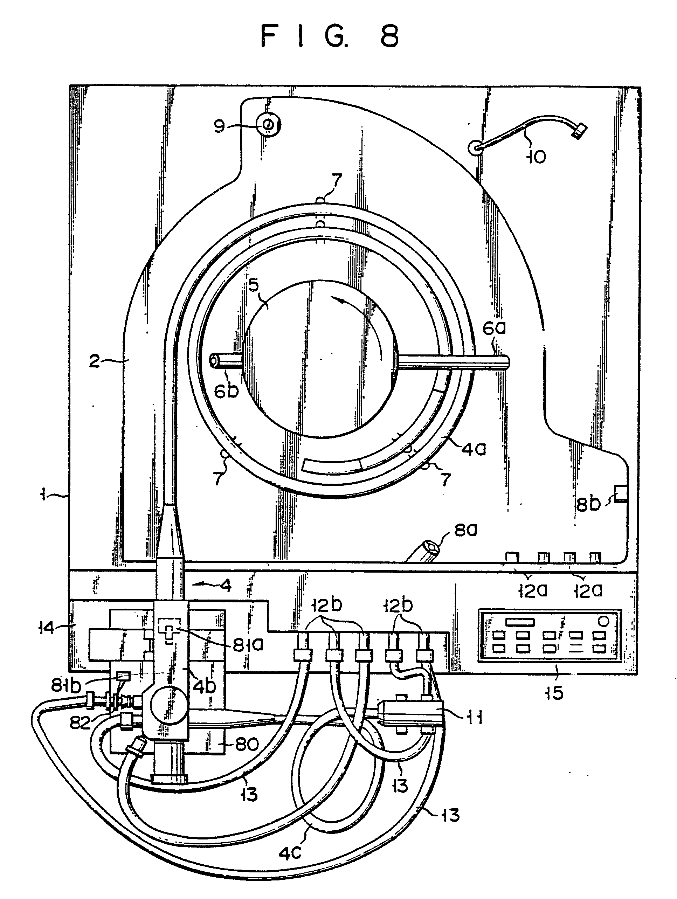

The front end portion of the upper surface of the body 1 is not covered by the cover 3 but is provided with recesses at its central and left-hand side portions. The left recess serves as a mounting portion 14 for placing the control section 4b thereon (Fig. 8) when an endoscope has a strcture such that only its insertion section is constructed water tight and may be subjected to washing. A semi-circular recess 14a is formed between the mounting portion 14 and the washing tank 2 so as to receive the distal end portion of the control section 4b therein. A plurality of supply ports 12b (five in this embodiment) are disposed at the side surface of the central recess. The washing liquid or the disinfectant is supplied to the supply ports 12b like the ports 12a. The ports 12b are used to wash or disinfect the endoscope, only its insertion section being constructed water tight. A main control panel 15 is arranged at the right-hand side of the front end portion of the upper surface of the body 1. A sub-control panel 16 is arranged below the main control panel 15 at the right-hand side of the front surface of the body 1.

A piping system which includes the pumps, tanks, and solenoid valves to supply a washing liquid, a disinfectant or compressed air will be described with reference to Fig. 3. These pumps, tanks, solenoid valves and the like are disposed below the washing tank 2 in the body 1. An air supplying means 19 will be described first. The air supplying means 19 mainly comprises the air pump 34 and the pipe 10. The air pump 34 is connected to the pipe 10 disposed inside the washing tank 2. A relief valve 23 is connected to a point midway along the pipe 10 between the port 11 and the air pump 34. The pressure of compressed air from the air pump 34 is then controlled by the relief valve 23. This compressed air is then supplied to the inside of the endoscope 4 through the port 11 and the pipe 10.

A washing means 18 comprises mainly a water tank 43, a water pump 35 and the nozzles 6 (the upper and lower nozzles 6a and 6b). Tap water is supplied from a faucet 47 to the water tank 43 through a filter 31 and a solenoid valve 24. A water level detector 43a is arranged in the water tank 43 to detect the level of water stored therein. When the detector 43a detects a lower limit of the level of water, the solenoid valve 24 is opened, so that more water is supplied from the faucet 47 to the water tank 43. When the water level detector 43a then detects an upper limit of the level of water, the solenoid valve 24 is closed, so that excess water is not supplied from the faucet 47.

The water pump 35 and a filter 32 are interposed at a pipe which connects the water tank 43 and the nozzles 6a and 6b. A washing agent tank 45 is connected to a point midway along the pipe connecting the water tank 43 and the water pump 35, through a series combination of an orifice 46 and a solenoid valve 25. The ports 12 (12a, 12b) which supply washing water to the four channels of the endoscope placed in the washing tank 2 are connected to a point midway along the pipe communicating the filter 32 with the nozzles 6 through a series combination of a check valve 39 and a mixing header 42. An air pump 33 is connected to the mixing header 42 through a check valve 41. A solenoid valve 26 is connected to a point midway along the pipe communicating the check valve 41 and the air pump 33 so as to be able to release the compressed air from the air pump 33. When the water pump 35 is operated, tap water from the water tank 43 is supplied to the nozzles 6 and the ports 12. In this case, when the solenoid valve 25 is opened, the washing agent from the washing agent tank 45 is mixed in the water from the water tank 43, thus obtaining a washing liquid. This washing liquid is supplied to the nozzles 6 and the ports 12. However, when the solenoid valve 26 is closed and the compressed air from the air pump 33 is supplied to the mixing header 42, bubbles are present in the washing liquid which is supplied from the water pump 35 to the mixing header 42. The washing liquid with bubbles present therein is supplied to the ports 12. When the elimination of bubbles from the washing liquid is required, the solenoid valve 26 is opened and compressed air is released from the air pump 33.

The water tank 43 is connected to another water pump 37 through a solenoid valve 28. The water pump 37 is connected to the inflow portion of a solenoid valve 29 for selecting the flow path. One end of the outflow portion of the solenoid valve 29 is connected to the washing tank 2, and the other end thereof is connected to the ports 12 through the mixing header 42. The water supplied from the water tank 43 upon operation of the water pump 37 is supplied to one of the ports 12 or the washing tank 2 in accordance with the operation of the solenoid valve 29.

A disinfecting means 20 mainly comprises the water pump 37 and a disinfectant tank 44. The disinfectant tank 44 is connected to the intake port of the water pump 37 through the solenoid valve 27. Therefore, when the solenoid valves 27 and 28 are opened and closed, respectively, the disinfectant is supplied from the disinfectant tank 44 to the washing tank 2 or the ports 12 upon operation of the water pump 37. When the float switch 9 arranged in the washing tank 2 detects that the level of the disinfectant or the water supplied to the washing tank 2 has reached a predetermined level, the water pump 37 is stopped.

A discharge port 17 is formed at the bottom of the washing tank 2. The inflow portion of a selector 30 is connected to the discharge port 17. One end of the outflow portion is connected to the disinfectant tank 44, and the other end thereof is connected to a water pump 36. The selector 30 is connected to a motor 22 through a rack and a pinion (not shown). Upon forward or reverse rotation of the motor 22, the selector 30 selects a predetermined flow path. When the selector 30 is switched to the side of the disinfectant tank 44, the disinfectant in the washing tank 2 returns to the disinfectant tank 44. However, when the selector 30 is switched to the side of the water pump 36, the water in the washing tank 2 is discharged by the water pump 36. When the inflow portion of the selector 30 does not communicate with any outflow end thereof, the water or the disinfectant in the washing tank 2 is kept therein.

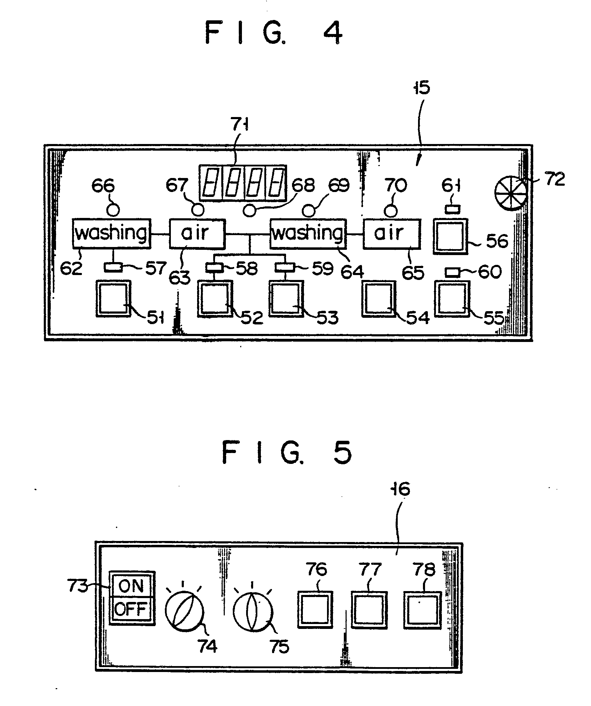

As shown in Fig. 4, the main control panel 15 has switches and display units which are frequently used. The switches are a "washing agent" switch 51, a "disinfectant" switch 52, an "overnight" switch 53, a "stop" switch 54, a "start" switch 55, and a "leakage" detection switch 56. Indicator lamps 57, 58, 59, 60 and 61 are arranged immediately above the switches 51, 52, 53, 55 and 56, respectively, so as to indicate their ON states. Cycle indicators 62 and 63 which respectively indicate washing by the water and air blowing (drying) in the washing cycle, and cycle indicators 64 and 65 which respectively indicate washing by the water and air blowing in the disinfectant cycle are arranged in the main control panel 15. The operations of these cycle indicators 62, 63, 64 and 65 are respectively indicated by indicator lamps 66, 67, 69 and 70 arranged immediately thereabove. The indicator lamp 68 is arranged to indicate the disinfection process. A four-digit LED display unit 71 is also arranged in the main control panel 15 to display the time remaining in the cycle or process. Furthermore, an emergency lamp 72 is arranged at the upper right corner of the main control panel 15 so as to indicate an emergency during operation. The sheet switches are used for switches 51, 52, 53, 54, 55 and 56 such that the surfaces of the switches do not project from the surface of the control panel. The indicators and the display unit do not project from the surface of the main control panel 15. The main control panel 15 thus has a flat surface as a whole.

Meanwhile, the sub-control panel 16 has a pushbutton type power (ON/OFF) switch 73; a dial 74 for setting a washing time and a dial 75 for setting a disinfecting time of a click dial type; and a manual "washing" switch 76, an "air" (drying) switch 77, and a "discharge" switch 78 (for discharging the disinfectant from the washing tank 2) of two-stroke type. Each of the switches 76, 77 and 78 is turned on upon a first stroke and is turned off upon a second stroke. The sub-control panel 16 has switches which are not frequently used. Therefore, even if the sub-control panel 16 is arranged on the upper right portion of the front surface, the operator will not be inconvenienced.

The operation of the apparatus having the structure described above will be described hereinafter.

When the endoscope 4 is constructed water tight as a whole, the insertion section 4a, the control section 4b and the light guide cable 4c are placed in the washing tank 2, as shown in Fig. 2. The respective channels of the endoscope 4 are connected to the ports 12a through the pipes 13, respectively. The distal end of the pipe 10 is connected to the connecting port 11 of the connector 4d of the light guide cable 4c. Thereafter, the cover 3 is kept opened, and the operator starts up the apparatus by turning on the power switch 73 on the sub-control panel 16.

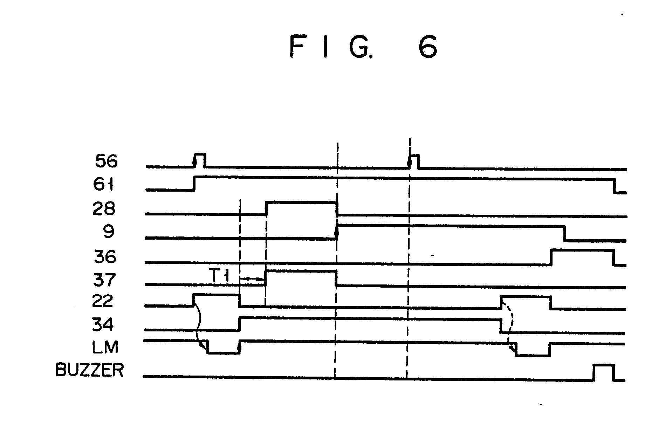

First, the operator performs detection of a leakage portion of the endoscope 4. As shown in the timing chart of Fig. 6, when the operator depresses the switch 56 in the main control panel 15, the indicator lamp 61 therein goes on. The leakage inspection mode is then set. The indicator lamp 61 continues to be ON until the leakage inspection mode is completed. At the same time, the motor 22 is operated to close the selector 30. The discharge port 17 of the washing tank 2 is closed. The operation of the selector 30 is properly controlled by a limit switch LM (see Fig. 6). Immediately after the discharge port 17 of the washing tank 2 is completely closed, the compressed air is supplied to the inside of the endoscope 4 through the pipe 10. Since the insides of the sections 4a, 4b and the cable 4c of the endoscope 4 communicate with each other, the inner space thereof is kept at the same pressure. Meanwhile, when a predetermined time interval $T_1$ has elapsed after the air pump 34 is operated, the solenoid valve 29 is switched to the side of the washing tank 2. The solenoid valve 28 is then opened, and the water pump 37 is operated, so that water (for inspection) is supplied from the water tank 43 to the washing tank 2. A time lag corresponding to the time interval T1 is included between the start-up of the air pump 34 and that of the water pump 37 so as to increase the internal pressure of the endoscope 4 to a sufficient pressure, thereby preventing the entrance of water therein. When the level of water in the washing tank 2 reaches the predetermined level, the float switch 9 is started to stop the water pump 37 and to close the solenoid valve 28. When water is supplied to the washing tank 2 to the preselected level, the endoscope 4 as a whole is immersed in water. Since the inner space of the endoscope 4 is pressurized, bubbles will be observed at a leakage portion such as a pinhole, a tear or the like. The bubbles rise through the inspection water, so that the operator can observe them to find the leak.

When the switch 56 is depressed within a predetermined interval, or when the predetermined inspection interval has elapsed, the motor 22 is automatically driven to switch the selector 30 to connect the discharge port 17 with the water pump 36. When the selector 30 is switched, the water pump 36 is operated to discharge water from the washing tank 2. Although the air pump 34 is stopped, the pressurized state of the endoscope 4 is maintained for a short period of time. When the water is completely discharged from the washing tank 2, the end buzzer is operated to signal this state to the operator.

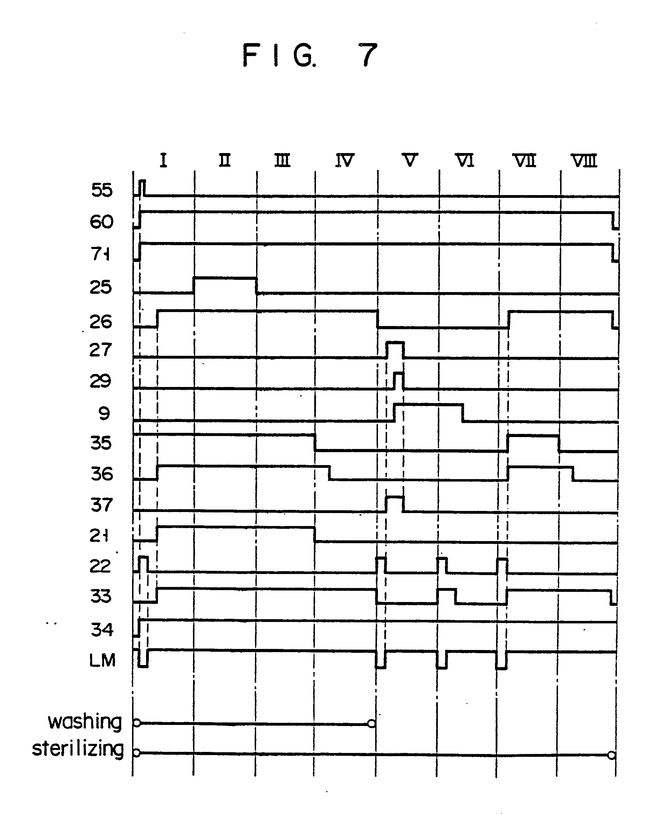

The washing cycle (using the washing liquid) and the disinfection cycle (using the disinfectant) will be described. If the operator wishes to perform only the washing cycle, only this cycle is performed. However, if he wishes to perform the disinfection cycle, the washing cycle is performed first, and then the disinfection cycle is performed. A case will be described in which the operator wishes to perform the disinfection cycle and depresses the disinfection switch 52. As shown in the timing chart of Fig. 7, when the operator depresses the start switch 55, its indicator lamp 60 goes on, and remaining disinfection time is indicated at the display unit 71. Subsequently, the motor 22 is started to switch the selector 30 to the discharge side. Immediately thereafter, the water pumps 35 and 36 are operated. At the same time, the motor 21 is driven, so that the nozzles 6a and 6b are rotated. The solenoid valve 26 is also closed.

In the washing cycle or the disinfection cycle, the air pump 34 is started to supply the compressed air into the endoscope 4 in the same manner as in the leakage inspection. This state is maintained during the washing cycle and the disinfection cycle. Even if a tear or a pinhole is formed at part of the endoscope 4 and is brought into contact with the liquid, the liquid will not penetrate into the inside of the endoscope 4, thereby preventing damage to the endoscope 4.

The washing liquid from the water tank 43 and the compressed air from the air pump 33 are mixed in the mixing header 42, and a resultant mixture is supplied to the channels respectively connected to the ports 12a through the pipes 13. The mixture then effectively washes inside the channels. Meanwhile, the washing liquid is supplied from the water tank 43 to the rotating nozzles 6a and 6b by means of the water pump 35. The water is sprayed from the rotating nozzles 6a and 6b to clean the outer surface of the endoscope 4 in the washing tank 2. If the operator had depressed th switch 51 before he depressed the start switch 55, the solenoid valve 25 is opened for a predetermined time interval corresponding to cleaning time preset by the dial 74 while the water is supplied. The washing agent is thus supplied from the washing agent tank 45 through the orifice 46, so that washing liquid with the washing agent is supplied to clean the endoscope 4. When washing with the washing agent is completed, only the solenoid valve 25 is closed to perform rinsing for a predetermined time by supplying water from the water tank 43 alone. When this is completed, the water pump 35 is stopped, so that no water is supplied any longer. At the same time, the motor 21 is stopped, so that the nozzles 6a and 6b stop rotating. However, the air pump 33 continues to supply the compressed air to the inside of channels of the endoscope 4 for a predetermined time interval so as to discharge the water remaining inside the channels of the endoscope 4. It is noted that the water pump 36 is operated until the water is completely discharged from the washing tank 2 and that the water pump 36 is then automatically stopped. Thereafter, the air pump 33 is stopped, and the solenoid valve 26 is automatically opened. In this manner, the washing cycle is completed. When the operator wishes to perform only the washing cycle without depressing the switch 52, the apparatus as a whole is stopped.

However, when the disinfection cycle is selected, this cycle is started. More particularly, the motor 22 is started to close the selector 30. The solenoid valve 27 is opened, and the water pump 37 is started to supply the disinfectant from the disinfectant tank 44 to the washing tank 2. When the disinfectant is supplied to the washing tank 2 so as to sufficiently immerse the endoscope 4 therein, the float switch 9 detects its level. The solenoid valve 29 is switched in response to the detection signal from the float switch 9, so that the disinfectant flows into the channels of the endoscope 4. When the channels are filled with the disinfectant, the solenoid valve 27 is closed and the water pump 37 is stopped. In other words, the outside of the endoscope and the inside of the channels thereof are disinfected by the disinfectant.

When a predetermined time interval has elapsed, the disinfectant is recovered in the disinfectant tank 44. More particularly, the motor 22 is started to switch the selector 30 which then communicates with the disinfectant tank 44, thereby flowing the disinfectant back to the disinfectant tank 44. Simultaneously as the motor 22 is started, the air pump 33 is started to discharge the disinfectant from the channels of the endoscope 4.

When the recovery of the disinfectant is completed, the motor 22 is started to switch the selector 30 which communicates with the discharge port 17. The water pump 36 is then started. At the same time, the solenoid valve 26 is closed, and the air pump 33 is started. Furthermore, upon operation of the water pump 35, the nozzles 6a and 6b spray the outer surface of the endoscope 4 with water without the washing agent, and the channels are cleaned with water with bubbles in the same manner as in the rinsing cycle, thereby cleaning the endoscope 4 as a whole. When this rinsing cycle is completed, the water pump 35 is stopped while the air pump 33 continues to operate. The water in the washing tank 2 is then completely discharged. In this manner, the washing cycle and the disinfection cycle are completed. It is possible to manually perform the washing cycle and drying cycle.

The endoscope 4 as a whole can be washed in the embodiment. However, the apparatus can be applied to an endoscope having a structure in which only its part (e.g., the insertion section) can be washed and disinfected.

As shown in Fig. 8, an endoscope 4 having a structure in which only its insertion section 4a can be washed and disinfected is placed in the washing tank 2. The insertion section 4a is placed on a holder 80 of the mounting portion 14. The channels of the endoscope 4 are respectively connected to the ports 12b through the pipes 13 at the outside of the washing tank 2. Detection switches 81a and 81b are arranged on the holder 80. When the control section 4b of the endoscope 4 is located in position, the detection switches 81a and 81b are operated to signal that the endoscope 4 has been set in position. For example, the detection switch 81a is arranged to be brought into contact with the outer surface of the control section 4b. The detection switch 81b is operated when a piston 82 properly urges an air/water supply changeover button of the endoscope 4. As previously described with reference to Fig. 2, an endoscope which is entirely liquid-tight may be completely placed in the washing tank 2. In this case, the detection switches 81a and 81b are not operated. In other words, the detection switches 81a and 81b serve as a detecting means for detecting the type of endoscope to be washed and disinfected.

As described above, the endoscope is immersed in inspection water while the compressed air is supplied to the inside of the endoscope through the corresponding pipe. The operator can detect a leakage portion by observing bubbles which will be formed if there is a pinhole or a tear. The damaged endoscope can be readily repaired, thereby preventing complete damage to the endoscope. Complete damage may be caused when the operator does not notice damage in an endoscope and when the endoscope is washed or disinfected. Furthermore, according to the apparatus of the present invention, the compressed air can be supplied during the washing and disinfection cycles. Therefore, even if a leakage portion is present in the endoscope, the washing liquid or the like will not penetrate into the endoscope, thereby preventing damage to the endoscope.

**Claims**

1. A washing apparatus for an endoscope, comprising a washing tank (2) adapted to receive a portion to be washed of an endoscope (4) therein; and washing means (18) for supplying a washing liquid to said endoscope (4) placed in said washing tank (2) so as to wash said endoscope (4) having an internal space, characterized by further comprising air supplying means (19) adapted to communicate with the internal space of said endoscope (4) placed in said washing tank (2), said air supplying means (19) being operable to supply air to said internal space.

2. An apparatus according to claim 1, characterized in that said washing means (18) has a washing water tank (43) for storing washing water, nozzles (6) connected to said washing water tank (43) to spray with the washing water the endoscope (4) in the washing tank (2) and a washing pump (35) disposed between the nozzles (6) and said washing water tank (43) to supply the washing water therefrom to the nozzles (6).

3. An apparatus according to claim 2, characterized in that said washing means (18) further has supply ports (12) connected to the washing pump (35), and connecting pipes (13) for communicating said supply ports (12) with channels of the endoscope (4), the washing water being supplied to said channels of the endoscope (4) through the supply ports (12) and the connecting pipes (13).

4. An apparatus according to claim 3, characterized in that said washing means (18) further has bubbling means (33, 42) for mixing air into the washing liquid supplied to said supply port (12) so as to form bubbles in the washing liquid.

5. An apparatus according to claim 4, characterized in that said bubbling means (33, 42) has a mixing header (42) disposed between said supply ports (12) and the washing pump (35), and a bubbling air pump (33) for supplying the air to the mixing header (42), the washing water supplied from the pump (35) to the supply ports (12) being mixed by the mixing header (42) with the air supplied from the air pump (33).

6. An apparatus according to claim 2, characterized in that said washing means (18) has washing agent supplying means (45, 25, 46) for supplying a washing agent into the washing liquid supplied from said washing water tank (43).

7. An apparatus according to claim 1, characterized by further comprising disinfecting means (20) for supplying a disinfectant to said endoscope (4) placed in said washing tank (2) to disinfect the endoscope (4).

8. An apparatus according to claim 7, characterized in that said disinfecting means (20) has a disinfectant tank (44) for storing the disinfectant, and a disinfecting pump (37) connected to said disinfectant tank (44) to supply the disinfectant to said washing tank (2), the disinfectant being stored in said washing tank (2) and said endoscope (4) being immersed in the disinfectant.

9. An apparatus according to claim 8, characterized in that said disinfecting means (20) has a first valve (29) an inflow end of which is connected to said disinfecting pump (37) and outflow ends of which are connected to said washing tank (2) and to said channel of the endoscope (4) placed in said washing tank (2), the disinfectant in the disinfectant tank (44) being supplied to said washing tank (2) or said channel of the endoscope (4) upon switching operation of the first valve (29).

10. An apparatus according to claim 9, characterized by further comprising a discharge port (17) disposed at a bottom of said washing tank (2) to discharge a liquid therein from the washing tank (2), a discharging pump (36) adapted to be connected to said discharge port (17), and a second valve (30) having an inflow end which is connected to the discharge port (17) and outflow ends which are connected to said disinfectant tank (44) and to the discharging pump (36), thereby recovering the disinfectant from the washing tank (2) to the disinfectant tank (44) or discharging the washing water by the discharging pump (36) from the washing tank (2), upon a switching operation of the second valve (30).

11. An apparatus according to claim 1, characterized in that said air supplying means (19) has an air supply pipe (10) adapted to communicate with said internal space of the endoscope (4), and an air pump (34) connected to the air supply pipe (10), thereby supplying compressed air to the internal space through the air supply pipe (10).

12. An apparatus according to claim 11, characterized in that the endoscope (4) has a light guide cable (4c) and a connector (4d) provided thereto for connecting the light guide cable (4c) and a light source, said connector (4d) having a connecting port (11) being connected to said air supply pipe (10), through which the compressed air from said air pump (34) being introduced into said internal space of the endoscope (4).

13. An apparatus according to claim 1, characterized by further comprising: liquid supplying means (43, 37) for supplying the liquid to said washing tank (2) to immerse the endoscope (4) placed in the washing tank (2) in the liquid, the air supplying means (19) supplying air to the internal space of the endoscope (4) immersed in the liquid.

**Patentansprüche**

1. Endoskop-Waschapparat, mit einem Waschbehälter (2) zur Aufnahme des zu waschenden Teiles des Endoskopes (4); und eine Waschvorrichtung (18) zum Zuführen einer Waschflüssigkeit zu dem Endoskop (4) in den Waschbehälter (2), so daß das Endoskop (4), welches einen Innenraum aufweist, gewaschen wird, gekennzeichnet durch eine Luftzufuhrvorrichtung (19), welche mit dem Innenraum des Endoskopes (4) in dem Waschbehälter (2) in Verbindung bringbar ist, wobei die Luftzufuhrvorrichtung (19) bei ihrer Betätigung Luft in den Innenraum einbringt.

2. Apparat nach Anspruch 1, dadurch gekennzeichnet, daß die Waschvorrichtung (18) einen Waschwasserbehälter (43) zur Aufnahme von Waschwasser, Düsen (6), welche mit dem Waschwasserbehälter (43) verbunden sind, um das Waschwasser auf das Endoskop (4) in dem Waschbehälter (2) zu sprühen und eine Waschpumpe (35) aufweist, welche zwischen den Düsen (6) und dem Waschwasserbehälter (43) angeordnet ist, um das Waschwasser aus dem Behälter den Düsen (6) zuzuführen.

3. Apparat nach Anspruch 2, dadurch gekennzeichnet, daß die Waschvorrichtung (18) Zufuhranschlüsse (12) aufweist, welche mit der Waschpumpe (35) in Verbindung stehen, sowie Verbindungsleitungen (13) aufweist, um die Zufuhranschlüsse (12) mit den Kanälen des Endoskops (4) zu verbunden, wobei das Waschwasser durch die Zufuhranschlüsse (12) und die Verbindungsleitungen (13) den Kanälen des Endoskopes (4) zugeführt wird.

4. Apparat nach Anspruch 3, dadurch gekennzeichnet, daß die Waschvorrichtung (18) weiterhin eine Bläschenvorrichtung (33, 42) aufweist, um Luft der Waschflüssigkeit unterzumischen, welche dem Zufuhranschluß (12) zugeführt wird, so daß Bläschen in der Waschflüssigkeit gebildet werden.

5. Apparat nach Anspruch 4, dadurch gekennzeichnet, daß die Bläschenvorrichtung (33, 42) einen Mischkopf (42) aufweist, der zwischen den Zufuhranschlüssen (12) und der Waschpumpe (25) angeordnet ist, sowie eine Luftpumpe (33) aufweist, um Luft dem Mischkopf (42) zuzuführen, wobei das Waschwasser, das von der Pumpe (35) den Zufuhranschlüssen (12) zugeführt wird in dem Mischkopf (42) mit der Luft von der Luftpumpe (33) vermischt wird.

6. Apparat nach Anspruch 2, dadurch gekennzeichnet, daß die Waschvorrichtung (18) eine Waschmittel-Zufuhrvorrichtung (45, 25, 46) aufweist, um ein Waschmittel der Waschflüssigkeit zuzufügen, welche von dem Waschwasserbehälter (43) gefördert wird.

7. Apparat nach Anspruch 1, weiterhin gekennzeichnet durch eine Desinfektionsvorrichtung (20) um ein Desinfektionsmittel dem Endoskop (4) in dem Waschbehälter (2) zuzuführen, um das Endoskop (4) zu desinfizieren.

8. Apparat nach Anspruch 7, dadurch gekennzeichnet, daß die Desinfektionsvorrichtung (20) einen Desinfektionsmittelbehälter (44) aufweist, um ein Desinfektionsmittel zu lagern, sowie eine Desinfektionsmittelpumpe (37) aufweist, welche mit dem Desinfektionsmittelbehälter (44) verbunden ist, um das Desinfektionsmittel dem Waschbehälter (2) zuzuführen, wobei das Desinfektionsmittel in dem Waschbehälter (2) eintritt und des Endoskop (4) in dem Desinfektionsmittel untergetaucht wird.

9. Apparat nach Anspruch 8, dadurch gekennzeichnet, daß die Desinfektionsvorrichtung (20) ein erstes Ventil (29) aufweist, dessen Einlaß mit der Desinfektionsmittelpumpe (37) verbunden ist und dessen Auslässe mit dem Waschbehälter (2) und dem Kanal des Endoskops (4) in dem Waschbehälter (2) verbunden sind, so daß das Desinfektionsmittel aus dem Desinfektionsmittelbehälter (44) bei einer Schaltbetätigung des ersten Ventils (29) entweder dem Waschbehälter (2) oder dem Kanal des Endoskops (4) zugeführt wird.

10. Apparat nach Anspruch 9, weiterhin gekennzeichnet durch einen Abfluß (17) an der Bodenfläche des Waschbehälters (2) um eine Flüssigkeit in dem Waschbehälter (2) abzulassen; durch eine Abflußpumpe (36) welche mit den Abfluß (17) verbunden ist; und ein zweites Ventil (30), dessen Einlaß mit dem Abfluß (17) verbunden ist und dessen Auslässe mit dem Desinfektionsmittelbehälter (44) und mit der Abflußpumpe (36) verbunden sind, so daß bei einer Schaltbetätigung des zweiten Ventils (30) Desinfektionsmittel aus dem Waschbehälter (2) in den Desinfektionsmittelbehälter (44) zurückgeführt wird oder das Waschwasser durch die Abflußpumpe (36) aus dem Waschbehälter (2) entfernt wird.

11. Apparat nach Anspruch 1, dadurch gekennzeichnet, daß die Luftzufuhrvorrichtung (19) eine Luftzufuhrleitung (10) aufweist, welche mit dem Innenraum des Endoskopes (4) in Verbindung bringbar ist, sowie eine Luftpumpe (34) welche mit der Luftzufuhrleitung (10) verbunden ist, so daß Druckluft in den Innenraum durch die Luftzufuhrleitung (10) gefördert wird.

12. Apparat nach Anspruch 11, dadurch gekennzeichnet, daß das Endoskop (4) einen Lichtleiter (4c) und ein Anschlußstück (4d) daran aufweist, um den Lichtleiter (4c) und eine Lichtquelle mit einander zu verbinden, wobei das Anschlußstück (4d) einen Verbindungsanschluß (11) aufweist, der mit der Luftzufuhrleitung (10) verbunden ist, so daß die Druckluft von der Luftpumpe (34) in den Innenraum des Endoskopes (4) eintreten kann.

13. Apparat nach Anspruch 1, weiterhin gekennzeichnet durch eine Flüssigkeitszufuhrvorrichtung (43, 37), um Flüssigkeit dem Waschbehälter (2) zuzuführen, so daß das Endoskop (4) in dem Waschbehälter (2) in der Flüssigkeit untertaucht, wobei die Luftzufuhrvorrichtung (19) luft in den Innenraum des Endoskopes (4), welches in der Flüssigkeit untergetaucht ist, fördert.

## Revendications

. 1. Appareil pour laver un endoscope, comprenant une cuve (2) de lavage prévue pour recevoir une partie à laver d'un endoscope (4); et un moyen (18) de lavage pour alimenter en liquide de lavage cet endoscope (4) placé dans la cuve (2) de lavage, de manière à laver l'endoscope (4) qui comprend un espace intérieur, caractérisé en ce qu'il comprend en outre un moyen (19) d'alimentation en air prévu pour communiquer avec l'espace intérieur de l'endoscope (4) placé dans la cuve (2) de lavage, ce moyen (19) d'alimentation en air pouvant être mis en oeuvre de façon à amener de l'air dans l'espace intérieur.

2. Appareil suivant la revendication 1, caractérisé en ce que le moyen (18) de lavage est doté d'un réservoir (43) d'eau de lavage pour stocker l'eau de lavage, de tuyères (6) reliées au réservoir (43) d'eau de lavage pour pulvériser d'eau de lavage l'endoscope (4) placé dans la cuve (2) de lavage, et une pompe (35) de lavage installée entre les tuyères (6) et le réservoir (43) d'eau de lavage pour alimenter les tuyères (6) en eau de lavage à partir de ce réservoir.

3. Appareil suivant la revendication 2, caractérisé en ce que le moyen (18) de lavage comprend en outre des orifices (12) d'alimentation reliés à la pompe (35) de lavage, et des tuyaux (13) de raccordement mettant en communication ces orifices (12) d'alimentation avec des conduits de l'endoscope (4), l'eau de lavage étant amenée à ces conduits de l'endoscope (4) par l'intermédiaire des orifices (12) d'alimentation et des tuyaux (13) de raccordement.

4. Appareil suivant la revendication 3, caractérisé en ce que le moyen (18) de lavage comprend en outre un moyen (33, 42) de bouillonnement pour créer un mélange d'air dans le liquide de lavage amené à l'orifice (12) d'alimentation de façon à former des bulles dans le liquide de lavage.

5. Appareil suivant la revendication 4, caractérisé en ce que le moyen (33, 42) de bouillonnement comprend un mélangeur-distributeur (42) interposé entre les orifices (12) d'alimentation et la pompe (35) de lavage, et une pompe (33) à air de bouillonnement pour alimenter en air le mélangeur distributeur (42), l'eau de lavage fournie par la pompe (35) aux orifices (12) d'alimentation étant mélangée par le mélangeur-distributeur (42) avec l'air fourni par la pompe (33) à air.

6. Appareil suivant la revendication 2, caractérisé en ce que le moyen (18) de lavage comprend des moyens (45, 25, 46) d'alimentation en agent de lavage fourni à partir du réservoir (43) d'eau de lavage.

7. Appareil suivant la revendication 1, caractérisé en ce qu'il comprend en outre un moyen (20) de désinfection pour alimenter en désinfectant l'endoscope (4) placé dans la cuve (2) de lavage, afin de désinfecter l'endoscope (4).

8. Appareil suivant la revendication 7, caractérisé en ce que ce moyen (20) désinfection comprend un réservoir (44) de désinfectant pour stocker le désinfectant, et une pompe (37) de désinfection branchée sur ce réservoir (44) de désinfectant pour alimenter en désinfectant la cuve (2) de lavage, le désinfectant étant stocké dans la cuve (2) de lavage et l'endoscope (4) étant immergé dans le désinfectant.

9. Appareil suivant la revendication 8, caractérisé en ce que le moyen (20) de désinfection est muni d'une première vanne (29) dont une extrémité d'admission est raccordée à la pompe (37) de désinfection et dont les extrémités d'évacuation sont raccordées à la cuve (2) de lavage et au conduit de l'endoscope (4) placé dans la cuve (2) de lavage, le désinfectant contenu dans le réservoir (44) de désinfectant étant amené à la cuve (2) de lavage ou au conduit de l'endoscope (4) par une manoeuvre de positionnement de la première vanne (29).

10. Appareil suivant la revendication 9, caractérisé en ce qu'il comprend en outre un orifice (17) de vidange disposé au fond de la cuve (2) de lavage pour évacuer le liquide de la cuve (2) de lavage, une pompe (36) de vidange pouvant se brancher sur l'orifice (17) de vidange, et une deuxième vanne (30) dont une extrémité d'admission est raccordée à l'orifice (17) de vidange et dont les extrémités d'évacuation sont raccordées au réservoir (44) de désinfectant et à la pompe (36) de vidange, ce qui permet de récupérer dans le réservoir (44) de désinfectant le désinfectant provenant de la cuve (2) de lavage ou d'évacuer l'eau de lavage par la pompe (36) de vidange de la cuve (2) de lavage par une manoeuvre de positionnement de la deuxième vanne (30).

11. Appareil suivant la revendication 1, caractérisé en ce que le moyen (19) d'alimentation en air comprend un tuyau (10) d'alimentation en air prévu pour communiquer avec l'espace intérieur de l'endoscope (4), et une pompe (34) à air raccordée à ce tuyau (10) d'alimentation en air, ce qui permet d'alimenter en air comprimé l'espace intérieur, par l'intermédiaire du tuyau (10) d'alimentation en air.

12. Appareil suivant la revendication 11, caractérisé en ce que l'endoscope (4) comporte un câble (4c) formant guide de lumière et un connecteur (4d) prévu pour connecter à une source de lumière le câble (4c) formant guide de lumière, ce connecteur (4d) comprenant un orifice (11) de connexion qui est relié au tuyau (10) d'alimentation en air par lequel l'air comprimé provenant de la pompe (34) à air est introduit dans l'espace intérieur de l'endoscope (4).

13. Appareil suivant la revendication 1, caractérisé en ce qu'il comprend en outre des moyens (43, 37) d'alimentation en liquide pour alimenter en liquide la cuve (2) de lavage dans le but d'immerger dans le liquide l'endoscope (4) placé dans cette cuve (2) de lavage, le moyen (19) d'alimentation en air amenant de l'air dans l'espace intérieur de l'endoscope (4) immergé dans le liquide.

F I G. 1

# F I G. 2

# F I G. 3

# F I G. 4

# F I G. 5

# F I G. 6

F I G. 7

# F I G. 8